# EUROPEAN PATENT SPECIFICATION

(11) **EP 2 958 616 B1**
(45) Date of publication and mention of the grant of the patent: **03.01.2024**
(21) Application number: 14753862.3
(22) Date of filing: 20.02.2014
(51) Int. Cl.: A61B 17/221, A61B 17/12

(54) **BLOOD FLOW RESTRICTION APPARATUS**
BLUTSTRÖMUNGSDROSSELUNGSVORRICHTUNG
APPAREIL POUR OBSTRUCTION DU FLUX SANGUIN

(30) Priority: 22.02.2013 US 201361768336 P
(43) Date of publication of application: 30.12.2015
(73) Proprietor: NeuroVasc Technologies, Inc., Irvine, CA 92618 (US)
(72) Inventor: MA, Jianlu, Irvine, CA 92618 (US)
(74) Representative: Prinz & Partner mbB
(86) International application number: PCT/US2014/017469
(87) International publication number: WO 2014/130716

(56) References cited:
- EP-A1- 1 452 142
- EP-A1- 1 867 290
- WO-A1-2012/162437
- WO-A1-2013/025531
- WO-A1-2015/057796
- WO-A2-03/094791
- WO-A2-2012/009675
- US-A- 5 941 869
- US-A1- 2011 009 941
- US-A1- 2011 130 784
- US-A1- 2011 251 629
- US-A1- 2011 264 132
- US-A1- 2012 083 868
- US-B2- 7 527 637
- US-B2- 8 313 503

## Description

This invention generally relates to devices useful for emboli retrieval and removal devices to treat, among other things, ischemic stroke and specifically to a mechanical thrombectomy device.

In particular, this invention relates to a mechanical thrombectomy device to retrieve and remove an obstruction responsible for a narrowing and/or blockage of the vessel(s) in neurovasculature or cardiac vasculature to restore oxygenated blood flow or superoxygenated blood distal of the blockage after the obstruction is being cleared.

### Background of the disclosure

This invention relates to medical mechanical thrombectomy devices and more particularly to collapsible and expandable devices for increasing blood flow through an obstructed blood vessel in neurovasculature and/or cardiac vasculature, this device can also be used to treat obstructed vessels in peripheral vasculature, such as in Deep Vein Thrombosis and related conditions, symptoms and disease states.

Currently, the FDA-approved treatment options for an acute ischemic stroke include intravenous (IV) delivery of clot dissolving medicine; and mechanical thrombectomy devices.

For treatment use clot dissolving medicine, the thrombolytic agent (Tissue Plasminogen Activator (t-PA)) is injected into the vasculature to dissolve blood clots that are blocking blood flow to the neurovasculature. Intravenous t-PA is currently limited in use because it must be used within a three hour window from the onset of a stroke and can result in an increased risk of bleeding. This standard of care leaves room for upgrading, lower aisle profiles and is only the appropriate approach to treatment for a limited class of individuals, groups and temporally-limited exigent cases.

The second option includes using mechanical thrombectomy devices. Such devices are designed to physically capture an embolus or clot and remove it from the blocked vessel, thereby restoring blood flow. The major advantage of the mechanical thrombectomy device is it can expand the treatment windows from 3 hours to over 10 hours.

Some existing mechanical thrombectomy devices used for increasing blood flow through an obstructed blood vessel include: 1 ) a filter trap designed and built to collect and remove emboli; 2) a cork-screwed guidewire like device to retrieve embolus; 3) a stent like device connected to a delivery wire to retrieve embolus. The major disadvantages of above mentioned existing mechanical thrombectomy devices include: 1 ) for filter like devices, filters tend to be cumbersome and difficult to delivery, deploy and a larger profile guide catheter may be needed to fully remove the embolus. In addition, it is difficult to coordinate precisely and predictably a desired movement to position the device properly in the vessel. The device can drift within the vessel, twist, or not be adequately conforming to the vessel wall and, therefore not effective for removing embolus; 2) for cork-screwed guidewire-like device, often they can only capture and remove embolus that is firm or is subject to certain mechanical variables such as being held together by itself as one piece. There is no immediate vascular re-canalization during the procedure and the existing stent like mechanical thrombectomy device is not capable of capturing small emboli that break off from the large embolus if any, and can lead to complications such as blockage of distal smaller vessels, vessel dissection, perforation and hemorrhage arise as a result of over-manipulation in the vessel.

A common disadvantage from the above mentioned existing devices include 1 ) the device may capture an embolus, but then lose grasp of it and migrate/deposit it incidentally in another area of the neurovasculature, creating the potential for a new stroke in a different part of the neurovasculature; 2) the device is not capable to capture the small embolus break off from the major embolus and prevent it from migrating to a more distal area of the neurovasculature; 3) the relative large device profile prevents it from treating the distal small diameter vessels.

US 2011/0264132 A1 discloses a catheter device with a base expandable member made of a mesh-like structure, and with an exterior covered by a cover member. The cover member can be fabricated by a braid or membrane structure and is affixed to the interior or exterior of the base member. The cover can be tacked to the base member at one or more points and can move axially or can be fixed to the base member. The cover member can be placed on a proximal portion of the base member and can cover more than 20 percent of the base member.

WO 2012/009675 A2 discloses retrieval systems for removing obstructions from a body lumen. A combination device can include a conventional stent-like structure associated with a downstream funnel. The funnel and the stent are operated separately by controlling devices in the handle of the combined system. The stent can be moved axially to be completely outside of the funnel or can be withdrawn into the funnel. The stent portion of the device is designed to remove all or at least pieces of an obstruction, which if loosened are then collected downstream in the funnel which has a smaller mesh size. The physician is able to manipulate the axial position of the stent and funnel completely independent of one another.

WO 2012/162437 A1 discloses a interventional medical device for retrieving an obstruction in a blood vessel comprising a delivery shaft and a capturing structure located at a distal end of the shaft including a plurality of struts. The struts at least partially enmesh with the obstruction such that movement of the capturing structure permits dislocation of the obstruction. An eversible cover has a fixed section affixed to the capturing structure and a free section extending in opposite direction from the fixed section. When the shaft is moved axially within the blood vessel, the eversible cover everts over the capturing structure.

Another disadvantage to existing mechanical thrombectomy devices is that they are built using two or more distinct pieces that require either joints or bonding between the delivery system and the treatment device. This connection of the pieces generally results in a weakness in the device that can result in an unintentional separation of the two pieces, possibly leaving the treatment device in the body during embolus retrieval. Also, the treatment portion of mechanical thrombectomy devices (particularly stent like devices) tend to be cut from tubing that is larger than the delivery system, thus making the treatment portion the limiting factor in terms of minimizing the compacted profile of the device, requiring larger access systems and greater delivery force to deliver the device.

Other flaws in the current mechanical thrombectomy designs include poor visibility/radiopacity, lack of variation in the delivery portion to enhance and improve deliverability, and lack of coatings or modified surface textures on the treatment portion to enhance embolus affinity, etc. In conclusion, there is a great need for improved devices, device systems, and methods for increasing blood flow through a blood vessel as described herein. None of the existing medical mechanical thrombectomy devices address all necessary needs to date.

### Summary of the Invention

According to the present invention, a mechanical thrombectomy device is provided as defined in claim 1. The device is made from a single piece of tubing of biocompatible material, including a flow block feature in the device body portion, a guidewire-like delivery portion and an expandable, treatment portion. The construction of the device from a single piece allows for a seamless transition from the delivery portion to the treatment portion, thus removing any joints or bonding of the two portions together as separate pieces. This improves the strength of the system as a whole and greatly reduces the possibility of the two parts unintentionally detaching from each other. Likewise, the distal treatment portion is cut from a piece of material the same size as the proximal delivery portion, allowing the device to be compacted to a similar size profile giving it delivery advantages including a lower delivery force required and requiring small access systems, and the treatment portion's surface can be altered to enhance embolus affinity by either coating with a substance or changing the texture by mechanical or chemical means.

The device includes an elongate member (proximal delivery portion) and an expandable member (distal treatment portion) fabricated from a single piece of super elastic or shape memory biocompatible material (tubing). The expandable member is configured to be inserted into a blood vessel and defines multiple spaces/openings in a wall of the expandable member. The expandable member generally has a compacted configuration for delivery and insertion into the target location of a blood vessel and an expanded configuration in which the expandable member to engage/receive embolus/clots with the multiple space/openings on it. The proximal portion/end of the expandable member has a flow block feature to block the blood flow when the device is expanded during the procedure.

The expandable member, also referred to as the treatment portion herein, includes a first component having a stent like structure with multiple space/openings in its wall to help engage the embolus/clot and establish structural integrity of the device.

The profile of the treatment portion is not "smooth". It contains "peaks" and "valleys" formed by the spaces/openings along the length. The major frame of the "peaks" and "valley" is formed by two or more "spines" in helix/spiral configuration. The "peaks", "valleys", and spiral "spines" help to improve the embolus affinity for better clot adhesion during procedure. The blood flow block feature can also be built into the device body (working length) area to block the flow during procedure. One example is to cover the "Valley" area in the device body, so that the blood flow cannot go through the device /vessel lumen when the device is expanded, which helps the device to engage the clot and prevent /reduce the clots break a part or being flush away to the distal vasculature

The strut(s) in the stent like structure in an embodiment form angles with the longitudinal axis of the device in the range from at least about 5 to approximately 175 degrees. The strut(s) can have twists along their longitudinal axes.

The treatment portion in another embodiment has a tapered distal section collecting small embolus break offs from major clot(s) and preventing them from migrating to a more distal area of the neurovasculature.

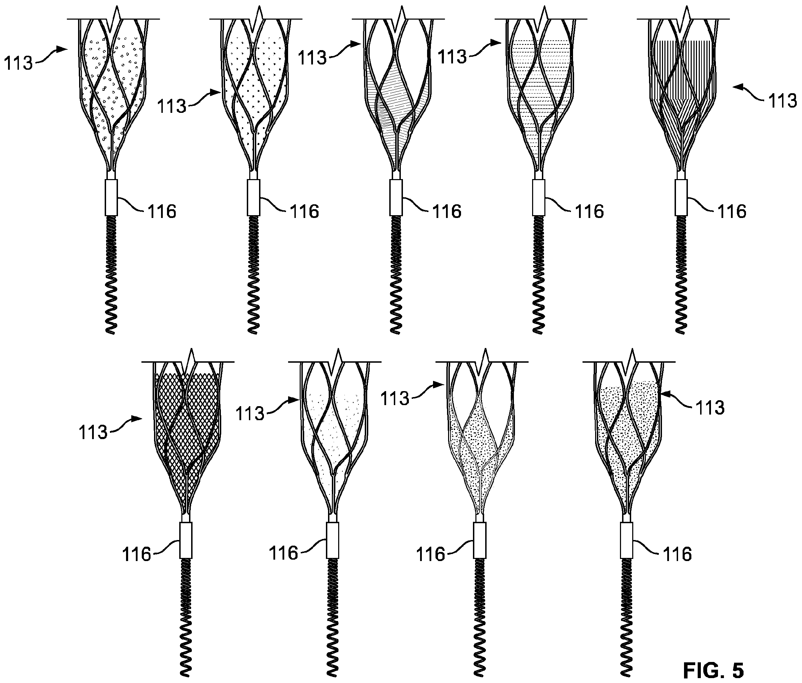

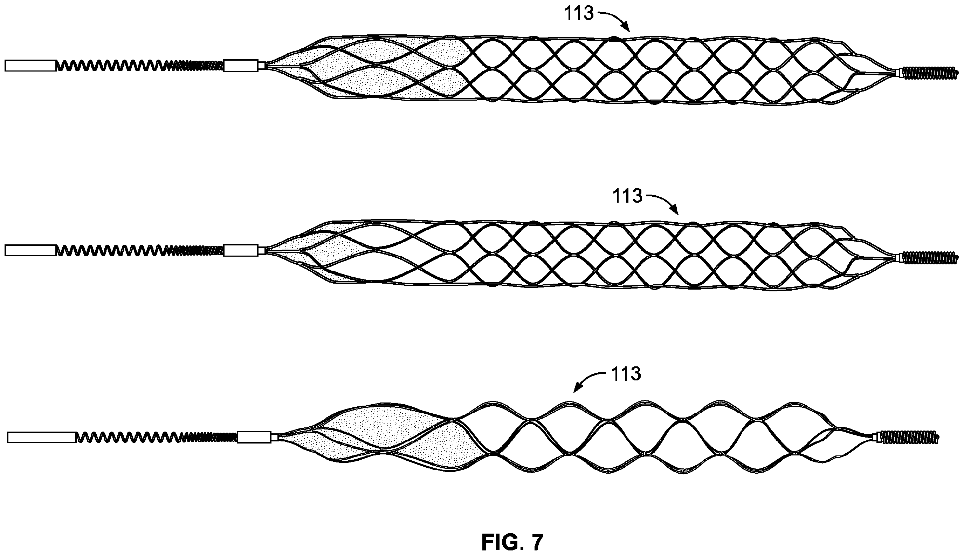

The device treatment portion has a flow block feature at its proximal portion to block the blood flow when device is expanded during the procedure. Figures 5 and 7 each shows some embodiments of the proximal flow block feature.

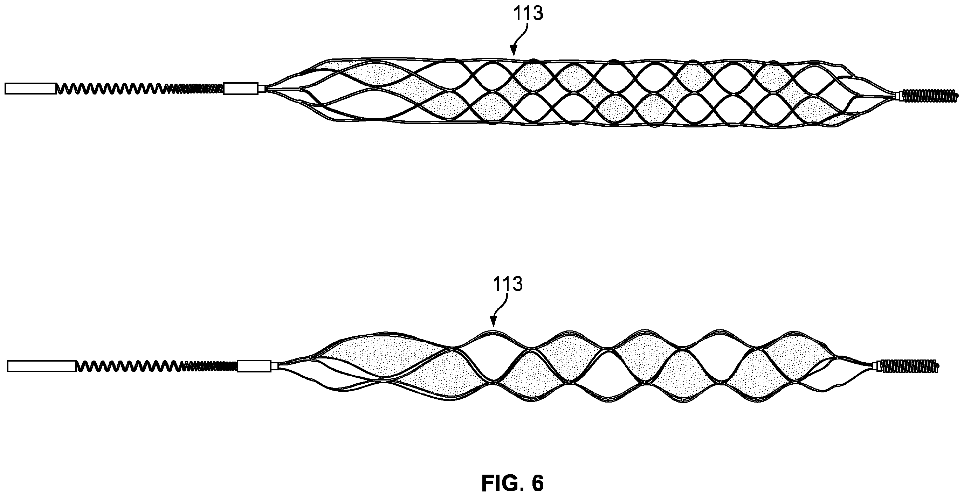

In another embodiment, the body of the treatment portion can have the flow block feature along the length. Figure 6 shows some exemplary configurations of the flow block feature in the expandable, treatment portion of the device.

The device can be made from either metallic biocompatible material (such as Nitinol, stainless steel, Co-Cr base alloy, Ta, Ti, etc.) or polymer based biocompatible material (polymers with shape memory effect, PTFE, HDPE, LDPE, Dacron, Polyester, etc.). For ischemic stroke treatment, the expandable stent-like member must be flexible enough to negotiate the torturous vasculature of the brain and without modifying the vessel profile at the target location. The profile of the expandable stent -like member must be small enough to reach target treatment site as known to artisans.

The expandable, treatment portion can be fully or partially coated with chemical(s), drug(s) or other bioagents to prevent clotting and/or for the better adhesion between the device and embolus. The device surface can be treated to form different surface layer (oxidation layer, Nitro -or carbonized or N-C-combined surface layer, etc.) for better adhesion between device and embolus. The device strut surface can be mechanically, chemically, or electrochemically treated to form "rough" surfaces for better adhesion between devices and emboli.

Radiopaque markers (marker coils, marker bands, Radiopaque wire(s), Radiopaque coatings, etc.) are integrated into the treatment device on the distal portion and proximal portion; or through the entire inner lumen of the treatment portion either partially or entirely to help position the device under standard fluoroscopy equipment.

In another embodiment, a transition portion of the device, where the proximal and distal portions meet will be seamless requiring no joints or bonding. Also, the transition portion can be modified with a number of variations to vary flexibility by having straight tubing, spiral cut through the wall thickness, or spiral cut partially through the wall thickness. When spiral cut, the flexibility can be varied through variable pitch sizes across the length. The transition portion can be covered by polymer tubing/layers/covers for the optimization of the device deliverability and the surface smoothness.

The inner lumen in the entire device can be used for the local drug delivery in the vasculature if needed. Following paragraphs describe the details of each embodiment of the device design.

### Brief Description of the Drawings

For a more complete understanding of the invention, reference is hereby made to embodiments as shown in the drawings, in which:
Figure 1 is an example of the overall profile of the device, according to an embodiment;
Figure 2 is an example of the distal portion (treatment portion) of another embodiment.
Figure 3a is an embodiment of the transition portion of the device with radiopaque material inserted into the lumen of the tubing and having larger dimensions at both ends (dumbbell shape), or can be attached onto the geometry.
Figure 3b is an embodiment of a transition portion of the device with a spiral cut through the entire wall thickness.
Figure 3c is an embodiment of a transition portion of the device with a spiral cut partially through the entire wall thickness.
Figure 4a is an embodiment of a transition portion of the device with a spiral cut configuration showing variable pitch sizes.
Figure 4b is an embodiment of a transition portion of the device with a spiral cut configuration through the entire wall thickness.
Figure 5 are embodiments of a proximal flow block feature on the proximal portion of the expandable, treatment portion.
Figure 6 shows embodiments of a flow block feature on the body portion of the expandable, treatment portion.
Figure 7 shows embodiments of the proximal flow block feature/el on the proximal portion of the expandable, treatment portion.

### Detailed Description of Embodiments

The present invention has numerous advantages.

A mechanical thrombectomy device is disclosed which is made from a single piece of biocompatible material, including a flow block feature in the device body portion, a guidewire like delivery portion and an expandable, treatment portion. The construction of the device from a single piece of tubing allows for a seamless transition from the delivery portion to the treatment portion, thus removing any joints or bonding of the two portions together as separate pieces. This improves the strength of the system as a whole and greatly reduces the possibility of the two parts unintentionally detaching from each other. Also, because the distal treatment portion is cut from a piece of material the same size as the proximal delivery portion, it allows the device to be compacted to a similar size profile giving it delivery advantages including a lower delivery force required and requiring small access systems. Additional delivery advantages from this design include the ability to manipulate the flexibility of the delivery system by varying the pitch size. In addition, a radiopaque marker can be attached within the lumen of the device to improve visualization. Lastly, the treatment portion's surface can be altered to enhance embolus affinity by either coating with a substance or changing the texture by mechanical or chemical means.

Compared with existing mechanical thrombectomy devices, the present, unique device design has the advantage of 1 ) having proximal flow block feature to block the blood distal flow when the device is deployed during use; this feature can help to eliminate or reduce the risk of flush or break the clots during the procedure; 2) the device may be made from a single piece of Nitinol super elastic material (such as tubing, etc.), Nitinol shape memory alloy material, or other biocompatible materials which exhibit super elastic or shape memory properties, thus giving the device a seamless transition from proximal delivery portion to distal therapeutic portion. This effectively removes any joints or bonding of a delivery wire with the treatment device, eliminating this physical weakness in the device and greatly reducing unintentional breakages during device delivery/retrieval. Another important advantage of the design in another embodiment is that various configurations (such as spiral cut, helix/coil configuration, etc.) can be implemented into the proximal delivery portion to achieve variable flexibility for easy delivery and navigation. The flexibility of the proximal delivery portion can vary from proximal to distal. For example, the distal portion can be more flexible than proximal portion. Furthermore, the device can achieve a smaller compacted profile, which reduces delivery and retrieval force and allows the physician to use smaller microcatheters for delivery to smaller vessels or the more distal vasculature. During procedure, this proximal flow block feature can block the blood flow through the lumen of the device and the lumen of the treatment vessel segment, to help engage the clot and eliminate or reduce the risk to break the clot or flush the clots distal to the more distal vasculature.

Detailed descriptions of the invention by way of embodiments are given in the following.

The present invention overcomes the shortcomings of the existing technologies and can be delivered to the target vasculature smoothly, retrieved safely, and remove the entire embolus. In use, the mechanical thrombectomy device can be compacted to a low profile and loaded onto a delivery system and delivered to the target location in the vessel by a medical procedure such as by use of a delivery catheter. The mechanical thrombectomy device can be released from the delivery system when it reaches the target implant site and recover to its normal expanded profile by the elastic energy stored in the device (self-expandable device).

As for the relative position of the device in relation to the embolus, it can either be deployed at the site of the embolus, or deployed distal to the embolus. In dealing with long embolus, the device can also be used to remove the embolus from the proximal portion to distal with multiple passes, until entire embolus is removed. The present invention offers the advantage of having a seamless transition from delivery portion to treatment portion from being fabricated from a single piece of biocompatible material tubing (which exhibits super elastic or shape memory properties, e.g. Nitinol). This feature dramatically reduces the possibility of an unintentional separation of the treatment device from the delivery wire.

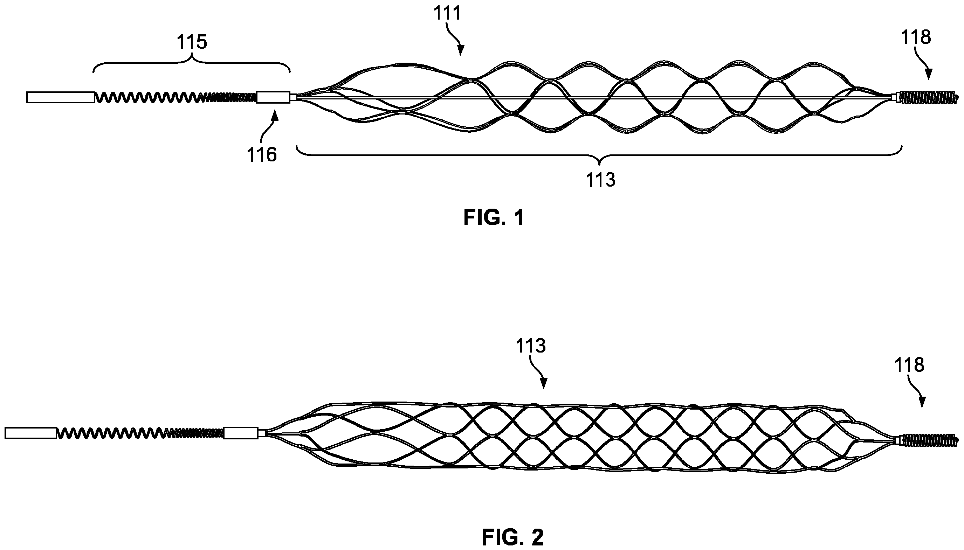

Turning now to the drawings, Fig. 1 and Fig. 2 each shows an embodiment of the overall profile of device 111. Device 111 can be made from one piece of Nitinol super elastic material or Nitinol shape memory alloy tubing. It is also made from other biocompatible materials that exhibit super elastic or shape memory properties. The device is made by laser cutting, mechanical machining, chemical machining, electrochemical machining, EDM, and related techniques known to artisans.

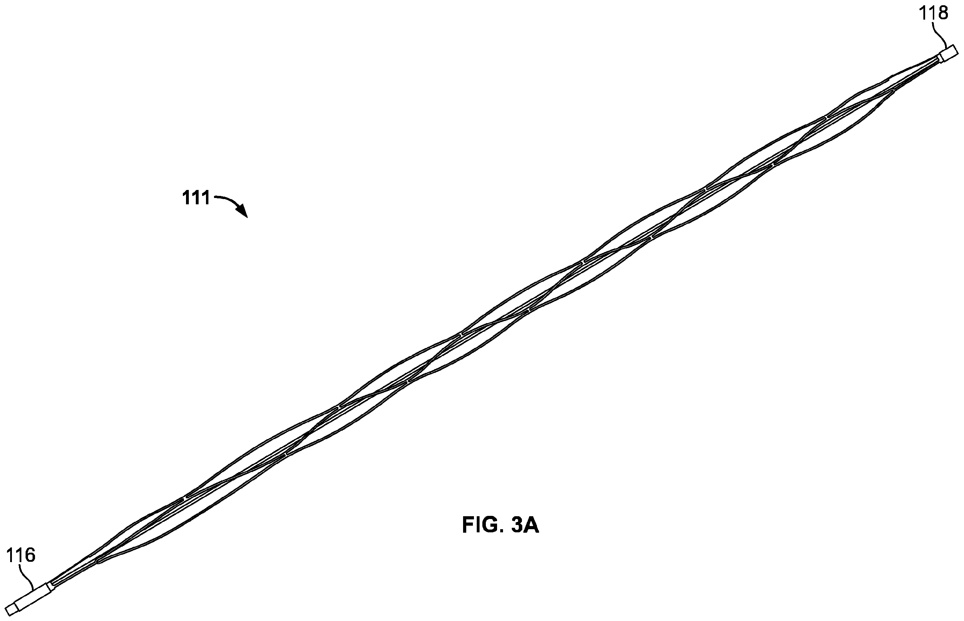

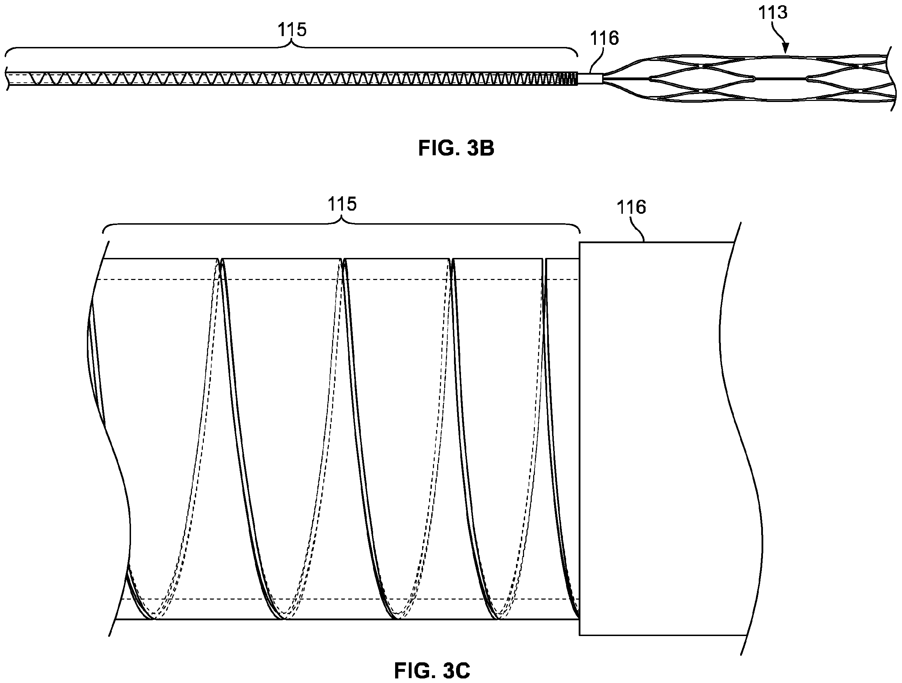

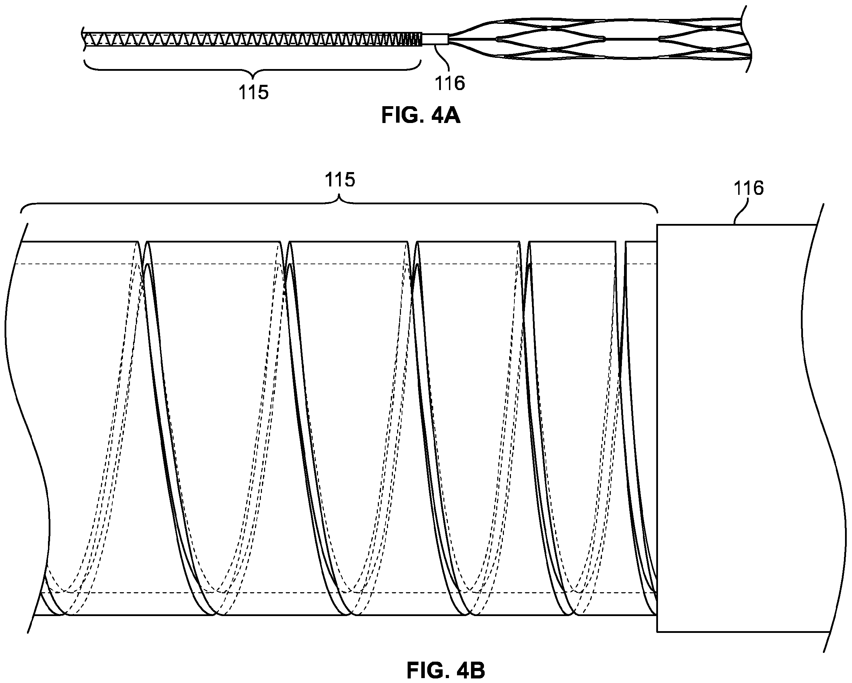

Treatment portion 113 is bordered on either end by proximal marker 116 and distal marker 118. Transition portion 115 is further detailed in Figures 3 and 4.

Figure 2 shows details of an embodiment with certain structures in treatment portion 113.

Figures 3A through 3C show examples of the transition portion 115 of the design. Transition portion can be 3A.) a straight piece of tubing; 3B.) a tubing with spiral cut through the entire wall thickness; 3C.) a tubing with spiral cut partially through the entire wall thickness. Other geometries can also be cut with an unlimited number of variations.

Figures 4A and 4B show examples of the transition portion 115 with spiral configurations. The pitch size can vary along the length for varying flexibilities. The spiral cut can either be through the entire wall thickness of the tubing or only partially through the wall thickness leaving a "groove" on the surface. In the case the spiral cut is through the entire wall thickness, the transition portion will have a real spiral profile (Figure 4B).

Figures 5 and 7 show the exemplary configurations of proximal flow block feature on the proximal portion of the expandable treatment portion.

Figure 6 shows the exemplary configurations of the flow block feature on the treatment portion 113 of the expandable portion.

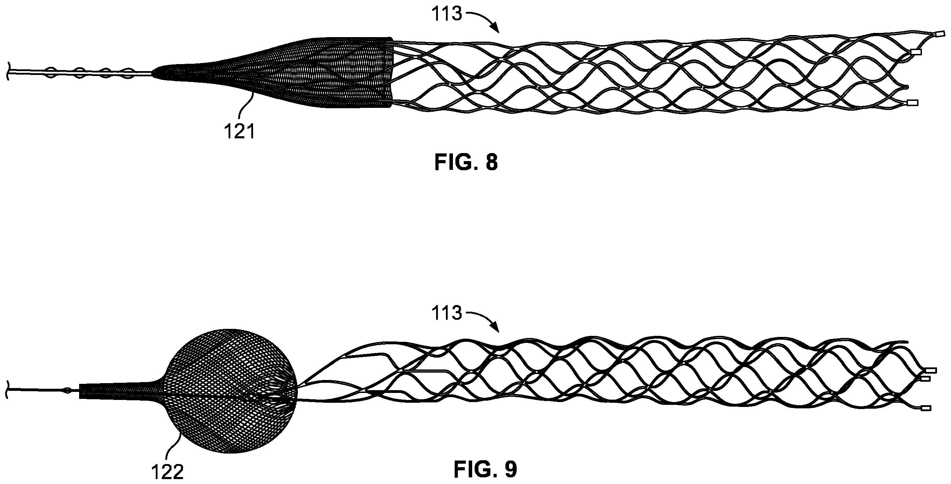

Figure 8 shows an embodiment in accordance with the present invention of the proximal flow restrictor with a braided wire tubular structure 121 from metallic or polymer materials.

Artisans readily understand that the proximal flow restrictor structure can be part or away from the proximal body of the device. The proximal flow block restrictor can have a first smaller compacted profile to make the delivery through microcatheter possible. The proximal flow restrictor can have a second larger expanded diameter/profile when the device is released from the microcatheter or other delivery system to block, limit, or restrict the blood flow.

Figure 9 shows an exemplary configuration of the proximal flow restrictor with a spherical or near spherical structure from metallic or polymer materials 122. The spherical structure can be braided or laser cut structure. It can be fabricated from the one or two element(s) of the device or fabricated from other pieces of material, then is attached onto the device proximal end by mechanical means, or thermal (laser or soldering) process, or adhesive/glue, or heat shrink technology.

The proximal flow restrictor structure can be part or away from the proximal body of the device. The proximal flow restrictor structure can have a first smaller compacted profile to make the delivery through microcatheter possible. The proximal flow restrictor can have a second larger expanded diameter/profile when the device is released from the microcatheter or other delivery system to block or limit, restrict the blood flow. One example is that the spherical or near spherical structure is made from braided metallic or polymer wires, then is attached onto the proximal portion of the device. One end (either proximal or distal end) of the spherical structure 122 can be loose or free to move, to accommodate the length change or variation during the delivery and expansion processes. The spherical structure can also be fabricated from the same piece of Nitinol tubing with that of the device by laser cutting or chemical processes and then shape set to a larger diameter than the raw Nitinol tubing.

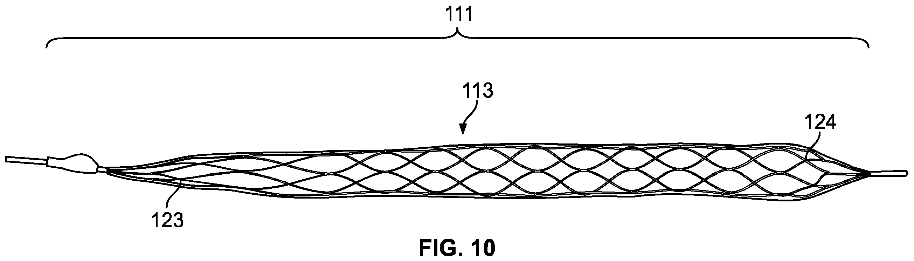

Figure 10 shows an exemplary configuration of a clot removal device 111 with flow block feature 113 and "wells" 123/124 in the cell space. The flow block feature described here can be made from polymer materials, the polymer metal can block the lumen of the device and also form "wells" or volume at each cell space to house the clot and prevent the clot to be break off or loose during the procedure.

The proximal portion design can be a straight tubing portion. The proximal flow block/restriction feature can be combined and used with any existing mechanical clot retriever to help remove the clot from vasculature.

Radiopaque markers can be attached on any portion of the device for positioning. One way to gain the full visibility for the device is to run a radiopaque material through the entire or partial lumen of the delivery wire. Markers can also be placed on the treatment portion to aid in positioning.

The device can have surface treatment on various portions to improve performance for the various portions of the device. The proximal and transition portion can either be coated or covered by typical biocompatible materials for lubricity entirely or partially. The surface of the distal treatment portion can have either a positive or negative charge for improved clot adhesion. The surface of the distal treatment portion can also be either mechanically or chemically treated to have a "rough" surface for improved clot adhesion. The "rough" surface can be achieved by 1) Porous surface coating or layer; 2) Micro blasted surface or micropinning; 3) Irregular strut geometry or arrangement.

It will be appreciated by those skilled in the art that changes could be made to the embodiments described without departing from the invention as defined in the appended claims.

## Claims

1. A mechanical thrombectomy device, comprising:
a guidewire-like delivery portion, an expandable treatment portion (113) having a body portion and a flow block element in the body portion, all made from a single piece of tubing of biocompatible material, so as to provide a seamless transition from the delivery portion to the treatment portion, and
a proximal flow restrictor (121) is attached onto the proximal end of the body portion, wherein the proximal flow restrictor (121), when in the expanded state, is adapted to have an expanded profile spaced a distance away from the proximal end of the treatment portion (113).

2. The device of claim 1, wherein the proximal flow restrictor (121) has a first smaller compacted profile, and a second larger expanded profile of near-spherical structure when the device is released from a microcatheter

3. The device of claim 2, wherein one end of the proximal flow restrictor (121) is free to move so as to accommodate changes in length or variation during the delivery and expansion of the proximal flow restrictor (121).

4. The device of claim 1, wherein a transition portion (115) of the tubing is provided where the guidewire-like delivery portion meets the treatment portion (113) to form the seamless transition, the transition portion (115) configured to have varying flexibility.

5. The device of claim 4, wherein the transition portion (115) can be a straight piece of tubing, a tubing with a spiral cut through the entire wall thickness or a tubing with a spiral cut through partially through the wall thickness.

6. The device of claim 1, 2 or 3, wherein the distal end of the body portion is tapered.

7. The device of claim 1, 2 or 3, wherein the proximal end of the body portion is tapered.

8. The device of claim 1, wherein a distal marker (118) is placed at the distal end of the body portion.

9. The device of claim 1, wherein a proximal marker (116) is placed at the proximal end of the body portion.

10. The device of any preceding claim, wherein the proximal flow restrictor (121) is a braided structure.

11. The device of any preceding claim, wherein the proximal flow restrictor (121) and the treatment portion (113) are made from a super elastic material or shape memory material.

12. The device of any preceding claim, wherein the proximal flow restrictor (121) extends along a length of about 1% to 50% of the length of the body of the treatment portion (113).

## Patentansprüche

1. Mechanische Thrombektomievorrichtung, mit:
einem führungsdrahtartigen Zuführabschnitt, einem ausdehnbaren Behandlungsabschnitt (113) mit einem Körperabschnitt und einem Strömungsblockierelement in dem Körperabschnitt, die alle aus einem einzigen Schlauchstück aus biokompatiblem Material hergestellt sind, damit ein nahtloser Übergang vom Zuführabschnitt zum Behandlungsabschnitt geschaffen ist, und
einem proximalen Strömungsbegrenzer (121), der am proximalen Ende des Körperabschnitts befestigt ist, wobei der proximale Strömungsbegrenzer (121) so ausgebildet ist, dass er im ausgedehnten Zustand ein ausgedehntes Profil aufweist, das in einem Abstand vom proximalen Ende des Behandlungsabschnitts (113) gelegen ist.

2. Vorrichtung nach Anspruch 1, wobei der proximale Strömungsbegrenzer (121) ein erstes kleineres komprimiertes Profil und ein zweites größeres ausgedehntes Profil mit beinahe kugelförmiger Struktur aufweist, wenn die Vorrichtung von einem Mikrokatheter gelöst ist.

3. Vorrichtung nach Anspruch 2, wobei sich ein Ende des proximalen Strömungsbegrenzers (121) frei bewegen kann, um Änderungen in der Länge oder Veränderungen während der Zuführung und Ausdehnung des proximalen Strömungsbegrenzers (121) Rechnung zu tragen.

4. Vorrichtung nach Anspruch 1, wobei ein Übergangsabschnitt (115) des Schlauchs dort vorgesehen ist, wo der führungsdrahtartige Zuführabschnitt auf den Behandlungsabschnitt (113) trifft, um den nahtlosen Übergang zu bilden, wobei der Übergangsabschnitt (115) mit unterschiedlicher Flexibilität ausgebildet ist.

5. Vorrichtung nach Anspruch 4, wobei der Übergangsabschnitt (115) ein gerades Schlauchstück, ein Schlauch mit einem spiralförmigen Schnitt durch die gesamte Wandstärke oder ein Schlauch mit einem spiralförmigen Schnitt durch einen Teil der Wandstärke sein kann.

6. Vorrichtung nach Anspruch 1, 2 oder 3, wobei das distale Ende des Körperabschnitts spitz zuläuft.

7. Vorrichtung nach Anspruch 1, 2 oder 3, wobei das proximale Ende des Körperabschnitts spitz zuläuft.

8. Vorrichtung nach Anspruch 1, wobei ein distaler Marker (118) am distalen Ende des Körperabschnitts angeordnet ist.

9. Vorrichtung nach Anspruch 1, wobei ein proximaler Marker (116) am proximalen Ende des Körperabschnitts angeordnet ist.

10. Vorrichtung nach einem vorhergehenden Anspruch, wobei der proximale Strömungsbegrenzer (121) eine geflochtene Struktur ist.

11. Vorrichtung nach einem vorhergehenden Anspruch, wobei der proximale Strömungsbegrenzer (121) und der Behandlungsabschnitt (113) aus einem superelastischen Material oder einem Formgedächtnismaterial bestehen.

12. Vorrichtung nach einem vorhergehenden Anspruch, wobei sich der proximale Strömungsbegrenzer (121) längs einer Länge von etwa 1% bis 50% der Länge des Körpers des Behandlungsabschnitts (113) erstreckt.

## Revendications

1. Dispositif mécanique de thrombectomie, comprenant :
un tronçon d'amenée de type fil de guidage, un tronçon de traitement extensible (113) qui présente un tronçon de corps et un élément de blocage d'écoulement dans le tronçon de corps, tous fabriqués à partir d'une seule pièce de tuyau en matière biocompatible de manière à fournir une transition ininterrompue du tronçon d'amenée au tronçon de traitement, et
un limitateur d'écoulement proximal (121) qui est attaché à l'extrémité proximale du tronçon de corps, le limitateur d'écoulement proximal (121), à l'état étendu, étant apte à présenter un profil étendu agencé à une distance de l'extrémité proximale du tronçon de traitement (113).

2. Dispositif selon la revendication 1, dans lequel le limitateur d'écoulement proximal (121) présente un premier profil compact plus petit et un deuxième profil étendu plus grand de structure quasiment sphérique lorsque le dispositif est détaché d'un micro-cathéter.

3. Dispositif selon la revendication 2, dans lequel une extrémité du limitateur d'écoulement proximal (121) est libre de se déplacer pour tenir compte de modifications en longueur ou de changements lors de l'amenée et de l'expansion du limitateur d'écoulement proximal (121).

4. Dispositif selon la revendication 1, dans lequel un tronçon de transition (115) du tuyau est prévu à l'endroit où le tronçon d'amenée de type fil de guidage se joint au tronçon de traitement (113) pour former la transition ininterrompue, le tronçon de transition (115) étant réalisé de manière à présenter une flexibilité variable.

5. Dispositif selon la revendication 4, dans lequel le tronçon de transition (115) peut être une pièce de tuyau droite, un tuyau présentant une coupure en spirale à travers l'entière épaisseur de paroi, ou un tuyau présentant une coupure en spirale à travers une partie de l'épaisseur de paroi.

6. Dispositif selon la revendication 1, 2 ou 3, dans lequel l'extrémité distale du tronçon de corps est effilée.

7. Dispositif selon la revendication 1, 2 ou 3, dans lequel l'extrémité proximale du tronçon de corps est effilée.

8. Dispositif selon la revendication 1, dans lequel un repère distal (118) est placé à l'extrémité distale du tronçon de corps.

9. Dispositif selon la revendication 1, dans lequel un repère proximal (116) est placé à l'extrémité proximale du tronçon de corps.

10. Dispositif selon l'une des revendications précédentes, dans lequel le limitateur d'écoulement proximal (121) est une structure tressée.

11. Dispositif selon l'une des revendications précédentes, dans lequel le limitateur d'écoulement proximal (121) et le tronçon de traitement (113) sont réalisés à partir d'un matériau superélastique ou d'un matériau à mémoire de forme.

12. Dispositif selon l'une des revendications précédentes, dans lequel le limitateur d'écoulement proximal (121) s'étend le long d'une longueur d'environ 1% à 50% de la longueur du corps du tronçon de traitement (113).
